# FASCICULE DE BREVET EUROPEEN

(11) **EP 1 976 589 B1**
(45) Date de publication et mention de la délivrance du brevet: **24.10.2018**
(21) Numéro de dépôt: 07731527.3
(22) Date de dépôt: 26.01.2007
(51) Int. Cl.: A61M 27/00

(54) **SYSTÈME DE DRAINAGE POUR LE TRAITEMENT DE L'HYDROCÉPHALIE**
DRAINAGESYSTEM ZUR BEHANDLUNG VON HYDROCEPHALUS
DRAINAGE SYSTEM FOR THE TREATMENT OF HYDROCEPHALY

(30) Priorité: 26.01.2006 FR 0650284
(43) Date de publication de la demande: 08.10.2008
(73) Titulaire: SOPHYSA, F-91893 Orsay Cedex (FR)
(72) Inventeur: NEGRE, Philippe, F-75116 Paris (FR); BONNAL, Olivier, 34212 Melsungel (DE)
(74) Mandataire: Nony
(86) Numéro de dépôt international: PCT/FR2007/050699
(87) Numéro de publication internationale: WO 2007/085771

(56) Documents cités:
- EP-A1- 0 115 973
- WO-A-01/54752
- DE-A1- 19 541 377
- FR-A1- 2 685 206
- US-A- 4 621 654
- US-A- 4 681 559
- US-B1- 6 689 085

## Description

La présente invention concerne un système de drainage pour le traitement de l'hydrocéphalie.

Certains patients porteurs d'une valve pour hydrocéphalie peuvent ressentir des symptômes d'hyperdrainage quand ils passent de la position couchée à la position assise ou debout. Ce phénomène, appelé « effet siphon », résulte de la pression hydrostatique, c'est-à-dire du poids de la colonne d'eau, qui s'instaure en position verticale entre l'extrémité proximale de la dérivation, au niveau cérébral et son extrémité distale, au niveau abdominal. La brusque variation de la pression intracrânienne, qui passe ainsi d'une valeur positive à une valeur fortement négative, provoque alors un drainage excessif du liquide céphalo-rachidien à travers la valve depuis les ventricules cérébraux vers le péritoine.

L'hyperdrainage est susceptible d'entraîner un certain nombre de complications, comme une réduction de la taille ventriculaire, l'apparition de ventricules fentes, la formation d'hématomes sous duraux ou d'hygromes.

De nombreux dispositifs ont été proposés pour lutter contre l'effet siphon :
- les dispositifs gravitationnels, qui utilisent le poids d'une ou plusieurs billes en tantale ou en acier pour augmenter la résistance de la valve lorsque le patient se redresse (valve Horizontal Vertical et dispositif GCA commercialisés par la société Integra, valve Chhabra Z-Flow commercialisée par la société Surgiwear, valve Dual-Switch, paedi-GAV, proGAv, Shunt Assistant commercialisées par la société Miethke). L'inconvénient de ces dispositifs est de ne pas fonctionner correctement s'ils sont déplacés de leur axe vertical après implantation ou si le patient s'adonne à des exercices physiques susceptibles de faire sauter la bille (jogging),
- les dispositifs à membrane de type Anti-Siphon Device (ASD) commercialisé par la société Heyer-Schulte, Siphon Control Device (SCD) ou Chambre Delta commercialisé par la société Medtronic PS Medical, reposant sur le déplacement d'une membrane en silicone lorsque la pression différentielle aux bornes de la valve devient inférieure à la pression atmosphérique. Ces dispositifs sont par conception particulièrement sensibles à la position d'implantation. Si le dispositif anti-siphon est implanté plus bas que l'extrémité d'entrée du cathéter ventriculaire, il y a risque d'hyperdrainage, alors qu'inversement, il y a risque d'hypodrainage si le dispositif est implanté plus haut que l'extrémité du cathéter ventriculaire. Ces dispositifs doivent également être en mesure de détecter la pression atmosphérique à travers la peau qui les recouvre. Une peau épaisse ou la formation d'un tissu cicatriciel au-dessus du dispositif sont susceptibles de l'inactiver. Ces dispositifs sont également très sensibles à toute augmentation de pression sous-cutanée, provoquée par exemple par l'appui de la tête sur un oreiller. Par ailleurs, ces dispositifs étant disposés en série par rapport à une valve à pression différentielle, leur résistance s'ajoute à celle de la valve dès lors que le patient se met en position verticale, ce qui conduit mécaniquement à une augmentation de la pression intracrânienne en position debout par rapport à la position couchée. Ces dispositifs fonctionnent donc de manière non physiologique, car chez un individu normal, on observe au contraire une baisse de la pression intracrânienne lors du passage de la position couchée à la position debout,
- les dispositifs à régulation de débit, de type Orbis Sigma. Ce type de dispositif présente une importante résistance hydrodynamique dans sa position de contrôle de débit en raison d'une section de passage réduite, ce qui le rend particulièrement sensible aux risques d'obstruction par des débris d'origine cérébrale ou des dépôts protéiques. Ce dispositif, conçu pour limiter le débit à la valeur moyenne de production du liquide céphalo-rachidien, soit 20 ml/heure, ne permet pas d'absorber sans augmentation excessive de la pression intracrânienne les importantes augmentations physiologiques de débit (jusqu'à 116 ml/h) provoquées pendant le sommeil par les ondes de pression vasogéniques cérébrales ou par les phases de mouvements oculaires rapides, ou encore engendrées par des quintes de toux. Ce système peut donc conduire à un hypodrainage, non seulement en position couchée, mais également en position debout quand la pression hydrostatique reste inférieure à la résistance de la valve, ce qui est le cas chez les jeunes enfants.

Le dispositif SiphonGuard™ commercialisé par la société Codman et décrit notamment dans le brevet US 6 126 628, est destiné à se connecter en série sur une valve à pression différentielle fixe ou programmable. Il comporte un passage principal sans résistance, contrôlé par une valve normalement ouverte, et un passage secondaire à haute résistance constitué d'un long trajet spiralé de faible diamètre. Quand le débit de drainage est faible, le liquide s'écoule à la fois par le passage principal et par le passage secondaire. Lorsque le débit dépasse un certain seuil, notamment quand le patient se lève, la valve se ferme et oblige le liquide à s'écouler par le passage secondaire à haute résistance, calibré pour limiter le débit. Un inconvénient est qu'en cas d'augmentation anormale de la pression intracrânienne, le dispositif devient alors incapable de s'ouvrir pour rétablir une pression normale dans les ventricules. Par ailleurs, la faible section du passage secondaire à haute résistance rend le dispositif particulièrement vulnérable aux obstructions par débris cérébraux ou dépôts protéiques, avec risque de blocage définitif du dispositif en position haute résistance par création d'un compartiment fermé entre la valve à pression différentielle et la valve obturatrice du trajet principal. Enfin, ce dispositif étant disposé en série, sa résistance vient s'ajouter à la valve à pression différentielle à laquelle il est relié. Cela signifie que le seuil de fermeture du trajet principal qui enclenche la limitation de débit ne peut pas être réglé indépendamment de la pression d'ouverture de la valve à pression différentielle. Il en résulte qu'un patient de grande taille avec une importante pression hydrostatique en position debout devra avoir une valve réglée en haute pression, au risque de présenter un hypodrainage en position allongée, et qu'un patient de petite taille, avec une faible pression hydrostatique devra avoir une valve réglée en basse pression, avec le risque de présenter un hyperdrainage en position allongée.

On connaît par le brevet US 5 336 166 un tel système comportant des première et deuxième valves à régulation de pression et une valve à régulation de débit. La première valve à régulation de pression est disposée en série avec l'ensemble formé des deux autres valves en parallèle. De manière alternative, la seconde valve à régulation de pression peut être disposée en parallèle avec un ensemble formé des deux autres valves en série. Ce système permet d'obtenir une pression de régulation constante ou un débit de régulation constant, en fonction des pressions appliquées au système. En particulier, entre le moment où la première valve à régulation de pression s'ouvre et le moment où la deuxième valve à régulation de pression s'ouvre, le débit de fluide céphalorachidien à travers le système de drainage reste sensiblement constant, ce qui correspond au fonctionnement thérapeutique du système. Lorsque ce système de drainage est configuré pour une régulation de débit à 5 ml/h et implanté sur un patient dont le débit de fluide est d'environ 20 ml/h, l'établissement d'une pression intercrânienne excessive au-delà de la zone de fonctionnement en régulation de débit peut causer certains symptômes et imposer l'implantation d'un nouveau système de drainage.

On connaît également par le brevet EP 688 575 une valve sous-cutanée permettant le réglage externe d'un passage ou d'une distribution de liquide, cette valve comportant un rotor et deux micro aimants montés sur celui-ci.

FR 2 685 206 divulgue une valve permettant une fuite.

EP 0 115 973 mentionne un débit de fuite compris entre 8 et 30 cm³/h.

L'invention a pour objet un dispositif de drainage selon la revendication 1.

Comme on le verra plus en détail dans la suite de la description, ce dispositif de drainage peut présenter au moins l'un des avantages suivants :
- permettre le réglage d'un niveau de pression en position debout indépendamment d'un niveau de pression en position allongée,
- permettre le fonctionnement du système indépendamment de la pression sub-cutanée, de la pression atmosphérique, de la position de la valve ou de la gravité,
- permettre de dissocier la fonction de régulation de pression en position allongée de celle en position debout, ces fonctions étant notamment assurées par deux mécanismes différents, indépendants l'un de l'autre, ce qui assure fiabilité et sécurité par rapport aux risques d'obstruction,
- permettre plusieurs niveaux de régulation de pression en fonction de la position du patient, notamment debout ou allongé,
- permettre le réglage, notamment par un médecin, d'un ou de plusieurs niveaux de pression, après implantation du dispositif sur le patient,
- limiter le risque d'obstruction du dispositif de drainage par des débris ou des dépôts organiques, et donc le risque d'avoir à réimplanter un nouveau dispositif de drainage sur le patient.

Enfin, l'obturateur et la restriction de passage sont conformés de manière à n'autoriser qu'une obturation partielle de la restriction de passage. Avantageusement, l'absence d'obturation totale permet de diminuer l'hystérésis entre l'ouverture et la fermeture partielle de cette restriction de passage.

De préférence, le dispositif de drainage selon l'invention comporte encore une ou plusieurs des caractéristiques optionnelles suivantes :
- Le débit minimum de sécurité est inférieur à 20 ml/h.
- L'obturateur et le bord de la restriction de passage sont conformés de manière à définir, dans la position d'obturation maximale, une section de passage entre l'obturateur et le bord de la restriction de passage telle que le débit minimum de sécurité, dans cette position, mesuré au moyen d'eau désionisée à 37 °C, soit supérieur à 2,5 ml/h et, de préférence, inférieur à 20 ml/h.
- De préférence, l'obturation progressive de la restriction de passage conduit à une diminution du débit et à une augmentation de la pression.
- De préférence, cette section de passage s'étend sensiblement perpendiculairement à la direction de déplacement de l'obturateur aux environs de la position d'obturation maximale. Ainsi, dans cette position, le fluide circule sensiblement parallèlement à la direction de déplacement de l'obturateur au passage de la restriction de passage. Il constitue ainsi un « matelas » de fluide entre l'obturateur et la restriction de passage particulièrement efficace pour diminuer l'hystérésis.
- Le premier siège et la restriction de passage sont de préférence, après réglage éventuel, fixes par rapport au corps définissant la chambre interne, l'obturateur étant mobile par rapport à ce corps.
- De préférence encore, l'obturateur présente une forme de révolution autour de son axe A de déplacement principal et est, de préférence, une bille. De préférence l'obturateur est mobile perpendiculairement à cet axe. Avantageusement, une telle conformation de l'obturateur permet son auto-centrage sous l'effet de l'écoulement du fluide, et donc une limitation des frottements avec le bord de la restriction de passage.
- Le dispositif peut comporter, avantageusement, un ressort de rappel agencé pour exercer sur une bille formant obturateur un effort tendant à l'appliquer sur le premier siège, la bille étant agencée pour pouvoir être déplacée vers ladite restriction de passage, à l'encontre de l'effort exercé par le ressort. De préférence, le ressort comporte au moins une portion de base tronconique s'élargissant vers l'extrémité inférieure du ressort et une portion supérieure cylindrique se raccordant à ladite portion de base tronconique.
- La chambre interne présente, en amont de la restriction de passage, la forme d'un tronc de cône s'évasant vers l'amont de l'écoulement et/ou se prolonge, en aval de la restriction de passage, par un tronc de cône s'évasant vers l'aval de l'écoulement, d'angle au sommet β supérieur à l'angle au sommet α du tronc de cône amont. Avantageusement, de manière surprenante, le « sablier » formé par les troncs de cônes amont et aval diminue l'hystérésis entre l'ouverture et la fermeture de la restriction de passage.
- Le dispositif de drainage est conformé de manière à ce que, lorsque la pression différentielle augmente, ledit obturateur ne commence à diminuer le débit à travers le dispositif que si ce débit est inférieur à 30 ml/h et/ou de manière à ce que la première pression d'ouverture soit inférieure à la pression différentielle initiale.
- Le dispositif de drainage comporte un port de dérivation communiquant avec la chambre interne, les ports d'entrée et de dérivation communicant entre eux et étant partiellement isolés du port de sortie par obturation de la restriction de passage par l'obturateur.
- L'obturateur et la restriction de passage sont conformés de manière à ce que l'obturateur puisse traverser, de préférence sans frottement, la restriction de passage lorsque la pression différentielle dépasse une pression d'ouverture, dite «à haute pression », supérieured'au moins 10%, à ladite pression de fermeture. De préférence, l'écartement entre l'obturateur et la restriction de passage, dans la position fermée d'obturation maximale de la restriction de passage par l'obturateur, est constant, c'est-à-dire que, dans ladite position fermée, la section de passage présente une largeur constante tout autour de l'obturateur. Avantageusement, le risque de frottements entre l'obturateur et la restriction de passage, et donc l'hystérésis entre l'ouverture et la fermeture de cette restriction de passage, en sont diminués.
- Le dispositif de drainage est conformé de manière à empêcher un débit de drainage supérieur à 20 ml/h si la pression différentielle ne dépasse pas ladite pression d'ouverture à haute pression.
   De préférence encore, le dispositif de drainage à obturation partielle selon l'invention est conformé de manière à ce que, lorsque la pression différentielle augmente, ledit obturateur ne commence à diminuer le débit à travers le deuxième siège de valve que si ce débit est inférieur à 30 ml/h.
   Avantageusement, il est ainsi possible de drainer du liquide céphalo-rachidien de manière conséquente même lorsque la pression différentielle est faible, pourvu qu'elle soit supérieure à la première pression d'ouverture. En position couchée du patient, on limite ainsi les risques d'un hypodrainage.
- De préférence, la première pression d'ouverture et/ou la pression de fermeture et/ou la pression d'ouverture à haute pression est ou sont réglables, ce(s) réglage(s) pouvant notamment être réalisé(s) sans contact, Selon un mode de réalisation avantageux, le(s) réglage(s) se fait (font) au moyen d'un couplage magnétique. Le dispositif comporte alors des moyens permettant un couplage magnétique avec l'extérieur du corps du patient pour effectuer le ou les réglages.
   Un réglage au moyen d'un moteur recevant ses instructions de commande et/ou son énergie par l'intermédiaire de radiofréquences est également possible. Avantageusement, le système de drainage peut ainsi être positionné à distance de la peau.
- Dans des variantes de l'invention, l'obturateur prend la forme d'une membrane ou d'une lame élastique aptes à obturer, en autorisant toujours cependant un débit minimum de sécurité, la restriction de passage.

Dans un mode de réalisation, la restriction de passage constitue un deuxième siège de valve sur lequel l'obturateur peut venir s'appliquer, l'obturateur et le deuxième siège de valve formant une deuxième valve pouvant se fermer, mais toujours en autorisant en service un débit minimum de sécurité, à une pression de fermeture prédéterminée supérieure, de préférence au moins 10 % supérieure, à la première pression d'ouverture.

Le dispositif de drainage peut comporter au moins une membrane élastiquement déformable avec au moins une portion définissant l'obturateur. En particulier, cette membrane peut comporter :
- un passage central agencé pour permettre le passage de fluide,
- des lèvres inférieure et supérieure, notamment annulaires, par exemple bordant sensiblement le passage central, la lèvre inférieure, respectivement supérieure, s'étendant sur une face inférieure, respectivement supérieure, de la membrane, la lèvre inférieure, respectivement supérieure, pouvant s'appliquer sur l'un des sièges de valve, respectivement l'autre des sièges de valve, en fonction de la déformation de la membrane, les lèvres définissant ainsi l'obturateur. Les lèvres inférieure et supérieure peuvent s'étendre à proximité ou de manière adjacente à un contour extérieur de la membrane. La lèvre réalisant la fermeture du dispositif de drainage à la pression de fermeture est conformée pour autoriser un débit minimum de sécurité dans la position fermée.

Alternativement, le dispositif de drainage peut comporter une lame élastiquement déformable comprenant une première portion maintenue de manière fixe dans la chambre interne et une deuxième portion mobile entre une première, respectivement une deuxième, position dans laquelle la lame s'applique contre le premier siège, respectivement le deuxième siège, la deuxième portion mobile de la lame définissant ainsi l'obturateur.

Le deuxième siège et la lame sont conformés de manière à autoriser un débit minimum de sécurité en position fermée.

On décrit également un système de drainage pour le traitement de l'hydrocéphalie, le système comportant :
- au moins un groupe de valves comportant :
   - une première valve à régulation de pression pouvant s'ouvrir à une première pression d'ouverture prédéterminée et permettre le passage de fluide,
   - une deuxième valve pouvant se fermer avec un débit minimum de sécurité, à une pression de fermeture prédéterminée supérieure à la première pression d'ouverture, les première et deuxième valves étant agencées de manière à ce que du fluide puisse être drainé à travers la deuxième valve lorsque la pression du fluide est comprise entre la première pression d'ouverture et la pression de fermeture,
- une troisième valve à régulation de pression pouvant s'ouvrir à une deuxième pression d'ouverture prédéterminée supérieure, de préférence au moins 10 %, de préférence encore au moins 20 %, supérieure à la pression de fermeture, les deuxième et troisième valves étant agencées de manière à ce que, lorsque la deuxième valve est fermée, en autorisant ledit débit minimum de sécurité, du fluide puisse être drainé à travers la troisième valve.

La troisième valve peut être une valve à régulation de pression réglable, par exemple de type POLARIS®, fabriquée par la société SOPHYSA (France).

Différents modes de réalisation sont envisageables. En particulier, les première et deuxième valves peuvent être disposées en série, la première valve étant en amont de la deuxième, et la troisième valve étant disposée en parallèle avec le groupe formé des première et deuxième valves.

Le système de drainage comporte un dispositif de drainage selon l'invention, l'obturateur dudit dispositif définissant avec le premier siège de valve et avec la restriction de passage, la première, respectivement la deuxième, valve du groupe de valves.

L'obturateur du dispositif de drainage pouvant traverser la restriction de passage, le dispositif peut également s'ouvrir sous l'effet de la pression d'ouverture « à haute pression » supérieure à la pression de fermeture. L'obturateur dudit dispositif peut alors définir, avec le premier siège de valve, la première valve, et avec la restriction de passage, les deuxième et troisième valves, la pression d'ouverture à haute pression constituant ladite deuxième pression d'ouverture de la troisième valve.

Les deuxième et troisième valves peuvent aussi être disposées en parallèle, la première valve étant disposée en série avec l'ensemble formé des deuxième et troisième valves, en amont de cet ensemble.

Le système de drainage peut également comporter une succession de groupes de valves formés chacun d'une première valve à régulation de pression et d'une deuxième valve, la première pression d'ouverture associée à la première valve d'un groupe donné étant supérieure à la pression de fermeture associée à la deuxième valve du groupe précédent. En particulier, il peut comporter au moins deux groupes de valves, les deux groupes de valves et la troisième valve étant disposés tous en parallèle. Chaque groupe peut comprendre un port d'entrée, un port de dérivation et un port de sortie, le port d'entrée d'un premier des groupes étant connecté au port de dérivation d'un deuxième des groupes de valves, et la troisième valve étant connectée au port de dérivation du premier groupe de valves.

Dans tous les modes de réalisation de l'invention décrits précédemment, l'obturateur ne peut obturer totalement ladite restriction de passage, c'est-à-dire que, dans la position fermée d'obturation maximale, la fermeture de la restriction de passage est partielle. Le débit à travers cette restriction, en position fermée, est alors supérieur à 2,5 ml/h. Dans la présente description, un débit supérieur à 2,5 ml/h est qualifié de « débit minimum de sécurité ».

On décrit également un système de drainage pour le traitement de l'hydrocéphalie, le système comportant :
- au moins un groupe de valves formant notamment un commutateur hydrostatique et comportant :
   - une première valve à régulation de pression pouvant s'ouvrir à une première pression d'ouverture prédéterminée et permettre le passage de fluide,
   - une deuxième valve pouvant se fermer à une pression de fermeture prédéterminée supérieure, notamment au moins 10 % ou 20 % supérieure, à la première pression d'ouverture, les première et deuxième valves étant agencées de manière à ce que du fluide puisse être drainé à travers la deuxième valve lorsque la pression du fluide est comprise entre la première pression d'ouverture et la pression de fermeture,
- une troisième valve à régulation de pression pouvant s'ouvrir à une deuxième pression d'ouverture prédéterminée supérieure, notamment au moins 10 % supérieure, à la pression de fermeture, les deuxième et troisième valves étant agencées de manière à ce que, lorsque la deuxième valve est fermée, du fluide puisse être drainé à travers la troisième valve.

L'une au moins des pressions d'ouverture et de fermeture est fixée par exemple lors de la fabrication de la valve correspondante.

La première pression d'ouverture est choisie avantageusement entre 30 et 130 mm H₂O, notamment entre 40 et 60 mm H₂O.

La pression de fermeture est choisie par exemple entre 80 et 120 mm. H₂O.

Dans un exemple de mise en oeuvre de l'invention, la différence entre la pression de fermeture et la première pression d'ouverture est inférieure à deux fois ladite première pression d'ouverture, notamment à une fois ladite première pression d'ouverture.

La première valve à régulation de pression permet un fonctionnement efficace du système de drainage lorsque le patient est en position allongée, avec un débit de drainage pouvant atteindre notamment au moins 20 ml/h, voire au moins 30 ml/h, lorsque la pression est entre la première pression d'ouverture et la pression de fermeture, tandis que la troisième valve à régulation de pression permet un fonctionnement efficace du système lorsque le patient est en position debout, avec un débit de drainage pouvant atteindre notamment au moins 20 ml/h, voire au moins 30 ml/h, lorsque la pression est supérieure à la deuxième pression d'ouverture.

Le système de drainage permet de s'affranchir d'un fonctionnement en régulation de débit, comme cela est le cas dans le système décrit dans le brevet US 5 336 166.

Le groupe de valves formant un commutateur hydrostatique selon l'invention est de préférence agencé de manière à ce que le débit de fluide drainé par ce groupe de valves soit sensiblement nul, avec un débit par exemple inférieur à 2,4 ml/h, lorsque la pression du fluide est supérieure à la pression de fermeture, à la différence par exemple du dispositif décrit dans le brevet US 6 126 628 qui présente un débit de drainage correspondant à une résistance constante lorsque la pression est supérieure à la pression de fermeture.

Dans un exemple de mise en oeuvre, les première et deuxième valves sont disposées en série, la première valve étant en amont de la deuxième, et la troisième valve est disposée en parallèle avec le groupe des première et deuxième valves formant commutateur hydrostatique.

Cette configuration permet de conserver une voie de drainage toujours libre, en cas d'obstruction de l'une des deux voies.

La troisième valve peut être une valve différentielle à haute pression.

Lorsque le patient est en position couchée, le fluide céphalo-rachidien s'écoule par la première valve à basse pression, qui offre le moins de résistance. Lorsque le patient se lève, il se produit une brusque dépression hydrostatique en aval de la première valve, en raison de la colonne de fluide formée entre l'extrémité d'un cathéter proximal et celle d'un cathéter distal. L'augmentation de la pression différentielle résultante provoque la fermeture de la deuxième valve, ce qui oblige alors le fluide à passer par le circuit parallèle à haute résistance constitué par la troisième valve à haute pression. L'ensemble peut ainsi constituer un commutateur hydrostatique automatique, activé uniquement par la variation de la pression différentielle régnant dans le circuit, et totalement indépendant de la pression sous-cutanée, de la pression atmosphérique, ou de la position de la valve par rapport au champ de la gravité terrestre.

En variante, les deuxième et troisième valves sont disposées en parallèle et la première valve est disposée en série avec l'ensemble formé des deuxième et troisième valve, en amont de cet ensemble.

Le groupe de valves formant commutateur hydrostatique peut ainsi être utilisé en dérivation d'une valve existante à pression différentielle, fixe ou programmable. L'ensemble constitue alors un système à résistance variable capable de commuter automatiquement d'une valve à régulation basse pression à une valve à régulation haute pression, et vice-versa, en fonction de la position du patient.

Le groupe de valves formant commutateur hydrostatique peut également être intégré dans un même boîtier à un mécanisme de valve, fixe ou programmable.

Avantageusement, la troisième valve est agencée de manière à ce que la deuxième pression d'ouverture qui lui est associée soit réglable, ce réglage pouvant notamment être réalisé sans contact.

La troisième valve peut par exemple être agencée pour permettre le réglage de la deuxième pression d'ouverture sur une valeur sélectionnée parmi N niveaux de pression, N étant un nombre entier.

Cette deuxième pression d'ouverture peut être réglée par exemple par le médecin en fonction de la taille du patient et/ou d'autres paramètres médicaux.

Le système de drainage peut être dépourvu d'aimant, notamment d'aimant permettant de régler depuis l'extérieur une pression d'ouverture du système de drainage.

L'absence d'aimant permet de supprimer le risque de déréglage du système de drainage lors du passage du patient dans des champs magnétiques forts tels que ceux générés par un dispositif d'imagerie à résonance magnétique nucléaire (RMN).

De plus, l'absence d'aimant permet de réaliser le système de drainage avec des matériaux amagnétiques tels que le plastique ou le phynox.

On peut notamment réaliser le système avec un coût réduit.

Dans un exemple de mise en oeuvre, le système comporte une succession de groupes de valves formés chacun d'une première valve à régulation de pression et d'une deuxième valve, la première pression d'ouverture associée à la première valve d'un groupe donné étant supérieure à la pression de fermeture associée à la deuxième valve du groupe précédent.

Une telle configuration permet d'avoir une gamme de niveaux prédéterminés de pressions d'ouverture et de fermeture, le système de drainage selon l'invention pouvant ainsi s'ajuster de manière automatique au type de fonctionnement, en fonction par exemple de la taille du patient et/ou de la position de celui-ci.

Cette configuration permet en outre d'éviter d'avoir à régler le système de drainage, avant ou après son implantation sur le patient, et également une sensibilité plus faible à l'obstruction d'une voie de drainage.

Le système peut comporter, le cas échéant, au moins deux groupes de valves, ces deux groupes de valves et la troisième valve étant disposés tous en parallèle.

Cette configuration permet de conserver une ou plusieurs voies de drainage toujours libres, en cas d'obstruction de l'une de ces voies.

En variante, le système comporte au moins deux groupes de valves, chaque groupe comprenant un port d'entrée, un port de dérivation et un port de sortie, le port d'entrée d'un premier des groupes étant connecté au port de dérivation d'un deuxième des groupes de valves, la troisième valve étant connectée au port de dérivation du premier groupe de valves.

Dans un exemple de mise en oeuvre, le groupe de valves comporte un dispositif de drainage comprenant :
- une chambre interne,
- au moins un port d'entrée et un port de sortie communiquant avec la chambre interne,
- au moins un obturateur disposé au moins partiellement dans la chambre interne,
- au moins des premier et deuxième sièges de valve,
l'obturateur définissant avec le premier, respectivement le deuxième, siège de valve, la première, respectivement la deuxième, valve du groupe de valves.

Le dispositif peut, le cas échéant, comporter un port de dérivation communiquant avec la chambre interne. Lorsque l'obturateur s'applique sur le deuxième siège, les ports d'entrée et de dérivation communiquent entre eux et sont isolés du port de sortie.

Le dispositif de drainage et la troisième valve peuvent être intégrés dans un boîtier commun ou, en variante, le dispositif de drainage peut être séparé de la troisième valve et relié à celle-ci par un conduit, notamment un conduit flexible tel qu'un conduit en élastomère.

Le conduit peut comporter par exemple une double lumière ou deux canaux.

Dans un exemple de mise en oeuvre de l'invention, l'obturateur comporte une bille et le dispositif comporte un organe de rappel élastique agencé pour exercer sur l'obturateur un effort tendant à l'appliquer sur le premier siège. L'obturateur peut être agencé pour pouvoir être déplacé vers le deuxième siège, à l'encontre de l'effort exercé par l'organe de rappel élastique.

Les sièges peuvent présenter par exemple une forme tronconique.

Avantageusement, l'organe de rappel élastique comporte un ressort, notamment un ressort hélicoïdal.

Le ressort est de préférence calibré à basse pression.

Le ressort peut comporter au moins une portion conique ou tronconique, et notamment une portion supérieure cylindrique se raccordant à une portion de base tronconique s'élargissant vers l'extrémité inférieure du ressort.

La bille peut ainsi être au contact d'une portion du ressort qui présente un diamètre relativement faible, tandis que la portion de base tronconique du ressort peut permettre, lorsque le ressort est comprimé par exemple lors du réglage des niveaux de pression d'ouverture et/ou de fermeture, un auto-centrage du ressort sur son support par accroissement du diamètre de la portion tronconique.

En outre, le diamètre relativement important de la portion de base tronconique peut permettre d'accroître la sensibilité du ressort et donc favoriser des déplacements de grande amplitude de l'obturateur. Il est ainsi possible de limiter le risque d'obstruction du port correspondant.

Dans un exemple de mise en oeuvre de l'invention, le dispositif comporte au moins une membrane élastiquement déformable avec au moins une portion définissant l'obturateur.

La membrane élastiquement déformable peut comporter par exemple :
- un passage central agencé pour permettre le passage de fluide,
- des lèvres inférieure et supérieure, notamment annulaires, bordant sensiblement le passage central, la lèvre inférieure, respectivement supérieure, s'étendant sur une face inférieure, respectivement supérieure, de la membrane. La lèvre inférieure, respectivement supérieure, peut s'appliquer sur l'un des sièges de valve, respectivement l'autre des sièges de valve, en fonction de la déformation de la membrane, les lèvres définissant ainsi l'obturateur.

En variante, la membrane élastiquement déformable peut comporter des lèvres inférieure et supérieure, notamment annulaires, s'étendant à proximité ou de manière adjacente à un contour extérieur de la membrane. La lèvre inférieure, respectivement supérieure, peut s'appliquer sur l'un des sièges de valve, respectivement l'autre des sièges de valve, en fonction de la déformation de la membrane, les lèvres définissant ainsi l'obturateur.

La membrane peut par exemple être réalisée en élastomère et présenter, le cas échéant, sensiblement une forme de disque.

Dans un autre exemple de mise en oeuvre de l'invention, le dispositif comporte une lame élastiquement déformable comprenant une première portion maintenue de manière fixe dans la chambre interne et une deuxième portion mobile entre une première, respectivement une deuxième, position dans laquelle la lame s'applique contre l'un des sièges de valve, respectivement l'autre des sièges, en fonction de la déformation de la lame, la portion mobile définissant ainsi l'obturateur.

La lame peut être en acier par exemple.

L'utilisation d'une lame élastiquement déformable peut permettre d'éviter certains inconvénients liés à la friction de l'obturateur sur une ou plusieurs parties du dispositif de drainage lorsque l'obturateur passe d'une position à une autre.

L'utilisation d'une lame permet en outre de limiter les risques d'obstruction des sièges de valve.

Avantageusement, les premier et deuxième sièges de valve sont réglables, notamment par déplacement de ceux-ci, de manière à pouvoir modifier la première pression d'ouverture et/ou la pression de fermeture associée au groupe de valves.

On décrit encore un dispositif de drainage, formant notamment commutateur hydrostatique, notamment pour un système de drainage tel que défini ci-dessus, comportant :
- une chambre interne,
- au moins un port d'entrée, un port de sortie, et optionnellement un port de dérivation, communiquant avec la chambre interne,
- au moins un obturateur disposé au moins partiellement dans la chambre interne,
- au moins un premier siège de valve associé au port d'entrée, l'obturateur pouvant s'appliquer sur le premier siège afin d'isoler le port d'entrée des ports de dérivation et de sortie, notamment lorsque la pression différentielle entre les ports d'entrée et de sortie est inférieure à une première pression d'ouverture prédéterminée,
- au moins un deuxième siège de valve associé au port de sortie, l'obturateur pouvant s'appliquer sur le deuxième siège afin d'isoler le port de sortie des ports d'entrée et de dérivation, notamment lorsque la pression différentielle entre les ports d'entrée et de sortie est supérieure à une pression de fermeture prédéterminée.

On décrit encore un dispositif de drainage utilisable notamment dans un système de drainage tel que défini ci-dessus, comportant :
- une chambre interne,
- au moins un port d'entrée, un port de sortie, et optionnellement un port de dérivation, communiquant avec la chambre interne,
- au moins un obturateur disposé au moins partiellement dans la chambre interne,
- au moins un premier siège de valve associé au port d'entrée, l'obturateur pouvant s'appliquer sur le premier siège afin d'isoler le port d'entrée des ports de dérivation et de sortie,
- au moins un deuxième siège de valve associé au port de sortie, l'obturateur pouvant s'appliquer sur le deuxième siège afin d'isoler, le port de sortie des ports d'entrée et de dérivation,
le dispositif étant caractérisé par le fait que l'obturateur comporte une bille, par le fait que le dispositif comporte un organe de rappel élastique agencé pour exercer sur l'obturateur un effort tendant à l'appliquer sur le premier siège, par le fait que l'obturateur est agencé pour pouvoir être déplacé vers le deuxième siège, à l'encontre de l'effort exercé par l'organe de rappel élastique, et, de préférence, par le fait que l'organe de rappel élastique comporte un ressort, comprenant notamment au moins une portion conique ou tronconique.

L'invention a encore pour objet un dispositif de drainage utilisable notamment dans le système de drainage tel que défini ci-dessus, comportant :
- une chambre interne,
- au moins un port d'entrée, optionnellement un port de dérivation et un port de sortie communiquant avec la chambre interne,
- au moins un obturateur disposé au moins partiellement dans la chambre interne,
- au moins un premier siège de valve associé au port d'entrée, l'obturateur pouvant s'appliquer sur le premier siège afin d'isoler le port d'entrée des ports de dérivation et de sortie,
- au moins un deuxième siège de valve associé au port de sortie, l'obturateur pouvant s'appliquer sur le deuxième siège afin d'isoler le port de sortie des ports d'entrée et de dérivation,
le système de drainage étant caractérisé par le fait qu'il comporte une lame élastiquement déformable comprenant une première portion maintenue de manière fixe dans la chambre interne et une deuxième portion mobile entre une première, respectivement une deuxième, position dans laquelle la lame s'applique contre le premier siège, respectivement le deuxième siège, la deuxième portion mobile de la lame définissant ainsi l'obturateur.

Dans la présente description, si dans la position, dite « fermée », d'obturation maximale de la restriction de passage par l'obturateur, il existe un débit minimum de sécurité entre l'obturateur et la restriction de passage. L'isolement ou la fermeture sont qualifiés de « partiels » (dispositifs à obturation partielle). Sinon, on considère que l'isolement est total ou que la fermeture est totale.

Par « restriction de passage », on désigne la partie de l'ouverture susceptible d'être obturée par l'obturateur et associée au port de sortie, et éventuellement à un port de dérivation, présentant une surface transversale minimale. La surface transversale est mesurée perpendiculairement à la direction générale de l'écoulement, en l'absence de l'obturateur. Dans le cas d'une ouverture en forme de sablier, la restriction de passage désigne donc le niveau d'étranglement maximal.

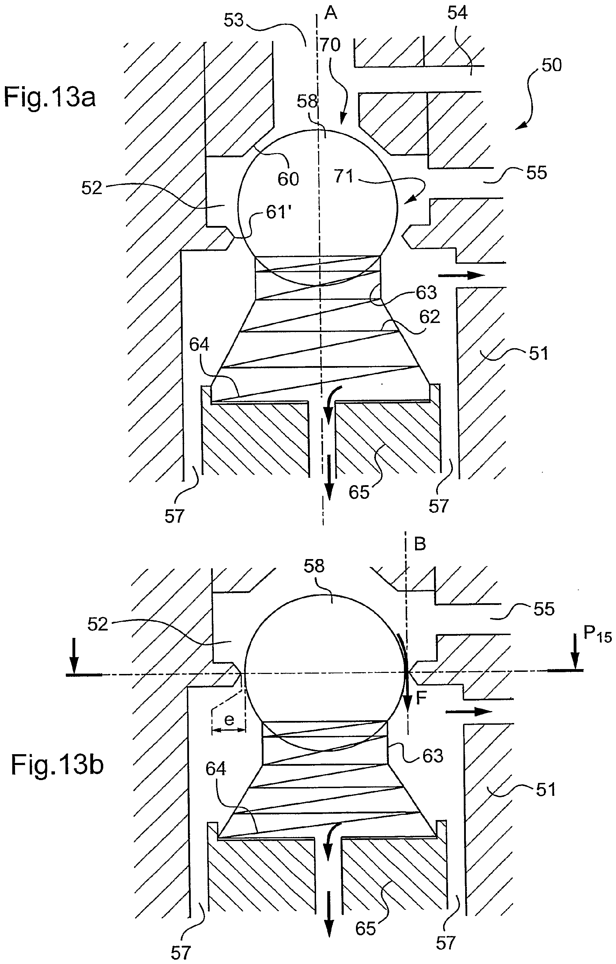

On considère que l'obturateur « traverse » la restriction de passage lorsque, par déplacement de l'obturateur depuis l'amont vers l'aval de cette restriction de passage, notamment sous l'effet d'une augmentation de la pression différentielle, la section de passage entre la restriction de passage et l'obturateur passe par un minimum, puis, après un éventuel maintien à une valeur minimale constante, augmente à nouveau. La section de passage minimale, de surface S, est obtenue dans la position d'obturation maximale de la restriction de passage par l'obturateur. L'obturation maximale commence lorsque la pression différentielle atteint une pression de fermeture P_{f} et est maintenue jusqu'à ce que la pression différentielle dépasse une pression d'ouverture « à haute pression » P_{HP}, Dans le cas particulier d'un obturateur se présentant sous la forme d'une bille, la bille « traverse » la restriction de passage lorsque son centre traverse le plan, noté P₁ sur la figure 13b, défini par la restriction de passage.

Sauf mentions contraires, tous les débits fournis dans la présente description sont relatifs à de l'eau désionisée à 37 °C. Cette eau est en effet un bon modèle du liquide céphalo-rachidien à drainer en service.

La « pression différentielle » est la différence de pression entre l'amont et l'aval du dispositif ou du système considéré.

La présente invention pourra être mieux comprise à la lecture de la description détaillée qui va suivre, d'exemples de mise en oeuvre non limitatifs de l'invention, et à l'examen du dessin annexé, sur lequel :
- les figures 1 à 4 représentent, schématiquement et partiellement, en coupe, différents exemples de dispositifs de drainage,
- la figure 5 est un graphique illustrant la variation de débit en fonction de la pression appliquée entre l'entrée et la sortie des dispositifs des figures 1 à 4,
- la figure 6 représente, schématiquement et partiellement, en coupe, une valve à régulation de pression,
- les figures 7 et 8 illustrent schématiquement différentes dispositions possibles des valves pour former un système de drainage,
- la figure 9 est un graphique illustrant la variation du débit en fonction de la pression appliquée aux systèmes des figures 7 et 8,
- les figures 10 et 11 illustrent schématiquement deux exemples de dispositions des valves afin de former des systèmes de drainage,
- la figure 12 est un graphique illustrant schématiquement la variation du débit en fonction de la pression appliquée aux systèmes de drainage des figures 10 et 11,
- les figures 13a à 13c représentent, schématiquement, en coupe, une variante d'un dispositif de drainage à obturateur traversant selon l'invention dans des positions correspondant à des pressions différentielles croissantes,
- la figure 14 représente une coupe schématique longitudinale d'une ouverture de deuxième valve d'une chambre interne d'un dispositif de drainage dans un mode de réalisation préféré,
- la figure 15 représente une coupe transversale selon le plan P₁₅ représenté sur la figure 13b, et
- les figures 16 et 17 représentent schématiquement, en coupe longitudinale, des variantes à membrane et à lame, respectivement, de dispositif de drainage à obturateur traversant selon l'invention.

Dans les différentes figures, des références identiques sont utilisées pour désigner des organes identiques ou analogues.

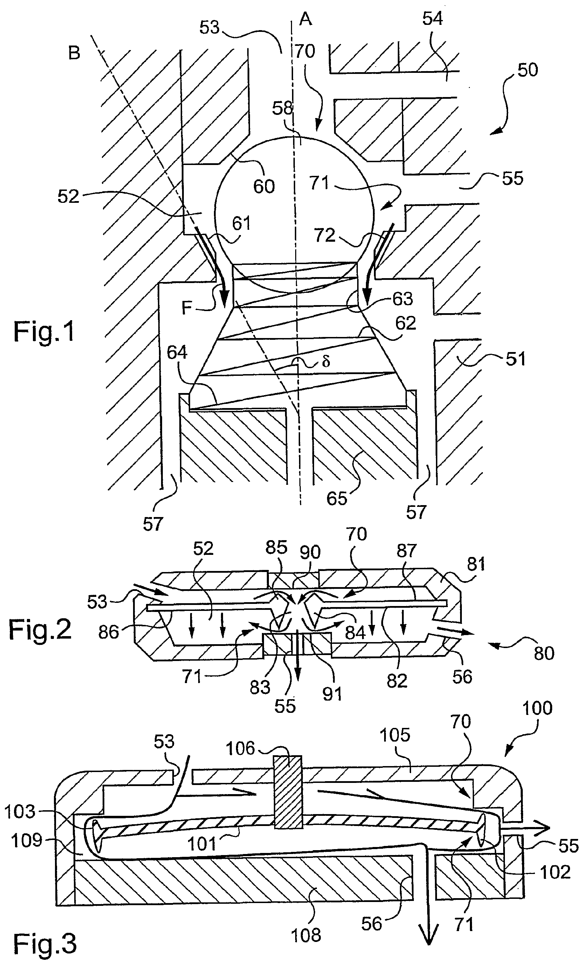

On a représenté sur la figure 1, un dispositif de drainage 50 comportant :
- un corps 51 définissant une chambre interne 52,
- un port d'entrée 53, des ports de dérivation 54 et 55 et des ports de sortie latéraux 57, ces ports communiquant avec la chambre interne 52,
- un obturateur 58 disposé dans la chambre interne 52, l'obturateur étant formé par une bille, par exemple en rubis ou tout autre matériau approprié,
- un premier siège de valve 60 associé au port d'entrée 53, la bille 58 pouvant s'appliquer sur ce premier siège 60 afin d'isoler le port d'entrée 53 du port de dérivation 55 et des ports de sortie 57,
- un deuxième siège de valve 61 associé aux ports de sortie 57, la bille 58 pouvant s'appliquer sur le deuxième siège 61 afin d'isoler, totalement ou partiellement, les ports de sortie 57 des ports d'entrée 53 et de dérivation 54 et 55,
- un organe de rappel élastique 62 formé par un ressort, agencé pour exercer sur la bille 58 un effort tendant à l'appliquer sur le premier siège 60, l'obturateur pouvant être déplacé vers le deuxième siège 61, à l'encontre de l'effort exercé par l'organe de rappel élastique 62.

Le deuxième siège 61 peut être réalisé sur une portion du corps 51 ou, en variante, être formé d'une rondelle assemblée avec le corps 51.

L'un au moins des sièges 60 et 61 peut être réalisé en métal, en rubis, en céramique ou en plastique, cette liste n'étant pas limitative.

Le ressort 62, notamment hélicoïdal, peut être métallique par exemple, et comporter, le cas échéant, au moins une portion ayant une forme cylindrique, conique ou tronconique.

Le ressort 62 peut comporter par exemple une portion supérieure cylindrique 63 se raccordant à une portion inférieure tronconique 64.

Le ressort 62 peut encore être du type plat-spiralé par exemple.

Le ressort 62 est posé sur un support 65 pouvant être réglé en hauteur afin de contrôler le niveau de compression du ressort 62.

La bille 58 définit avec le premier siège de valve 60 une première valve à régulation de pression 70 pouvant s'ouvrir à une première pression d'ouverture prédéterminée P₁ et permettre le passage de fluide.

Le premier siège de valve 60 peut être agencé, le cas échéant, pour être déplaçable par coulissement afin de régler la première pression d'ouverture P₁ à une valeur souhaitée.

La bille 58 définit avec le deuxième siège de valve 61 une deuxième valve 71 pouvant se fermer à une pression de fermeture prédéterminée P_{f} supérieure à la première pression d'ouverture P_{f}. Cette fermeture peut être totale ou, comme expliqué ci-après, peut être partielle.

Comme on peut le constater, les première et deuxième valves 70 et 71 permettent le drainage efficace de fluide céphalorachidien à travers la deuxième valve 71 lorsque la pression du fluide est comprise entre la première pression d'ouverture P₁ et la pression de fermeture P_{f}.

Le support 65 du ressort 62 étant réglable en hauteur, il est possible de régler la pression de fermeture P_{f} à une valeur souhaitée.

Par exemple, on peut avoir : (P_{f}- P₁) < 2P₁ ou (P_{f} - P₁) < P₁, par exemple P_{f} = 1,2 P₁.

La pression différentielle conduisant à l'application de la bille 58 sur le deuxième siège de valve 61 est inférieure à la pression différentielle nécessaire pour décoller la bille 58 de ce siège. En effet, lors de la fermeture de la valve, du fluide céphalorachidien circule autour de la bille 58, accélérant au passage du deuxième siège. Cette circulation crée donc une dépression en aval de la bille 58 qui favorise son déplacement vers le deuxième siège de valve 61. Lorsque la pression différentielle atteint une pression de fermeture P_{f} déterminée, la bille 58 entre en contact avec le siège de valve 61. Si la deuxième valve est à fermeture totale, la circulation du fluide céphalo-rachidien devient sensiblement nulle dans la position fermée d'obturation maximale du deuxième siège par l'obturateur. Si, par la suite, la pression différentielle diminue, le décollement de la bille 58 ne se produira pas à la pression P_{f}, mais à une pression plus faible, aucune circulation ne favorisant ce décollement. On appelle « hystérésis » un tel comportement asymétrique entre la fermeture et l'ouverture de la valve.

Les inventeurs ont découvert qu'il est avantageux de maintenir un débit minimum de sécurité à travers la deuxième valve dans la position d'obturation maximale de la valve. En particulier, il est avantageux que ce débit minimum de sécurité permette de maintenir un « matelas » de liquide céphalo-rachidien entre la bille 58 et le siège de valve 61. De préférence, les directions A et B sont sensiblement parallèles (figure 13b). Les inventeurs ont constaté que l'hystérésis en est considérablement diminuée.

Dans le mode de réalisation représenté sur la figure 1 où la bille 58 vient en contact avec le siège 61, des passages 72 peuvent être prévus pour permettre ce débit minimum de sécurité. Les inventeurs ont constaté qu'il était avantageux que le matelas d'eau résultant de la circulation du fluide céphalo-rachidien dans les passages 72 soit localement orienté selon l'axe A.

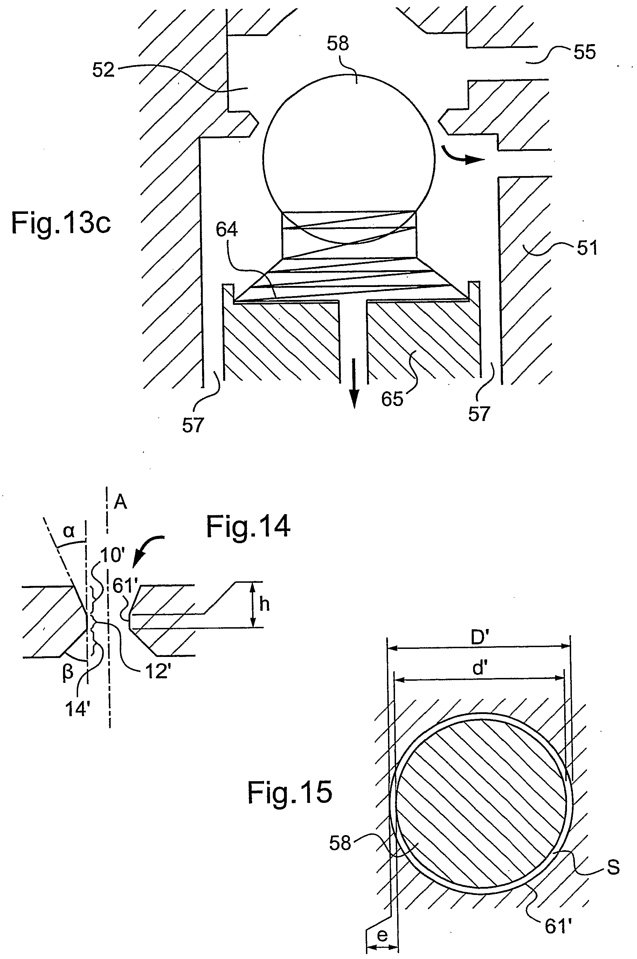

Dans un autre mode de réalisation selon l'invention, représenté partiellement sur les figures 13a à 13c, et sur la figure 14, la restriction de passage n'est pas un siège de valve, mais une ouverture 61' conformée de manière à pouvoir être traversée par l'obturateur, en l'occurrence la bille 58.

Comme représenté sur les figures 13b et 15, un écartement minimal constant « e » est prévu, dans la position d'obturation maximale représentée, entre la bille 58 et la restriction de passage 61'.

La restriction de passage 61' présente une forme circulaire de diamètre D'.

Cet agencement permet avantageusement de limiter le risque de frottements entre la bille 58 et la restriction de passage 61'. L'hystérésis entre l'ouverture et la fermeture (obturation maximale) de la restriction de passage en est avantageusement réduite.

Pour la même raison, la bille 58 est portée par le ressort 62 de manière à se déplacer, selon un axe de translation A passant par le centre de la bille 58, sensiblement perpendiculairement à la restriction de passage. Ainsi, quelle que soit la position de la bille 58, la circulation du fluide s'effectue de manière symétrique autour de l'axe A, ce qui permet un auto-centrage permanent de la bille 58 sur l'axe A. D'autant plus que la bille 58 est avantageusement portée par un ressort autorisant un léger déplacement transversal. Cette configuration limite avantageusement le risque de frottements avec la restriction de passage 61'.

Le mode de réalisation représenté sur les figures 13a à 13c est avantageux. En effet, il autorise, dans la position d'obturation maximale représentée sur la figure 13b, la création d'un matelas de fluide sensiblement cylindrique d'axe A entre l'obturateur et la restriction de passage. Un tel matelas est particulièrement avantageux pour diminuer l'hystérésis dans la position d'obturation maximale.

Dans la zone de la restriction de passage 61', le corps présente la forme d'un sablier, comme représenté en coupe longitudinale sur la figure 14. Depuis l'amont vers l'aval, cette zone présente une section tronconique amont 10', une section intermédiaire sensiblement cylindrique 12', de hauteur « h », et une section tronconique aval 14'. La section tronconique amont 10', divergeant vers l'amont, présente un angle au sommet α. La section tronconique aval 14', divergeant vers l'aval, présente un angle au sommet β.

Les troncs de cônes amont et aval se rejoignent au niveau de la section intermédiaire sensiblement cylindrique 12', coaxiale à l'axe A et de révolution autour de cet axe.

La section cylindrique 12', qui définit la restriction de passage 61' peut être d'une hauteur « h » sensiblement nulle (P_{f} = P_{HP}), comme représenté sur les figures 13a à 13c. Si elle est de hauteur non nulle (figure 14), la bille 58 peut maintenir un débit minimal à travers le deuxième siège de valve 61 tant que la pression différentielle évolue entre la pression de fermeture partielle P_{f} et une pression d'ouverture à haute pression P_{HP}.

La restriction de passage 61' est de préférence en rubis. Cette conformation diminue encore l'hystérésis.

Suite à une augmentation progressive de la pression différentielle P entre les ports d'entrée et de sortie, la bille 58 occupe les positions successives suivantes :
- Lorsque la pression différentielle est faible, la bille 58 s'applique de manière sensiblement étanche sur le premier siège 60 afin d'isoler le port d'entrée 53 du port de sortie 57. Cette position de fermeture est conservée jusqu'à ce que la pression différentielle atteigne une première pression d'ouverture P₁ prédéterminée.
- Au-delà de la première pression d'ouverture, la bille 58 occupe des positions successives (figure 13a) conduisant d'abord à une augmentation du débit D du fait d'un décollement de la bille 58 du premier siège de valve 60, puis, de préférence, à une diminution progressive de ce débit du fait d'une obturation croissante de la restriction de passage 61'. Le débit diminue jusqu'à ce que la pression différentielle atteigne une pression de fermeture P_{f} correspondant à une position d'obturation maximale de la restriction de passage 61' par la bille 58. Dans cette position, la section de passage à travers la restriction de passage 61' est minimale (figure 13b).

- Au-delà de la pression P_{f}, la bille 58 commence à traverser la restriction de passage 61'. Si la restriction de passage 61' présente une section de passage minimale sur une hauteur « h » non nulle (figure 14), le débit est maintenu à une valeur minimale tant que la pression différentielle reste inférieure à une pression d'ouverture à haute pression P_{HP}, supérieure à P_{f}.
- Quand la pression différentielle dépasse la pression de fermeture P_{f} ou, le cas échéant, la pression d'ouverture à haute pression P_{HP}, la bille 58 dégage progressivement la restriction de passage 61', en poursuivant sa course en aval de la restriction de passage (figure 13c). Le débit à travers le dispositif de drainage augmente de nouveau.

La pression d'ouverture à haute pression P_{HP} peut être définie de manière que la deuxième valve s'ouvre, en service normal, c'est-à-dire en dehors des phases de nettoyage.

Le dispositif de drainage selon l'invention peut ainsi avantageusement, à lui seul, constituer un système de drainage comportant :
- une chambre interne,
- au moins un port d'entrée 53, un port de sortie 57, et optionnellement un port de dérivation, communiquant avec la chambre interne,
- une bille 58 disposée dans la chambre interne,
- un premier siège de valve 60 associé au port d'entrée 53, la bille 58 pouvant s'appliquer sur le premier siège 60 afin d'isoler le port d'entrée 53 du port de sortie 57,
- une restriction de passage 61' associée au port de sortie 57, la bille 58 pouvant obturer partiellement ladite restriction de passage 61' afin d'isoler partiellement le port de sortie 57 du port d'entrée 53,
- un ressort 62 de rappel élastique agencé pour exercer sur la bille 58 un effort selon l'axe A tendant à l'appliquer sur le premier siège 60, et conformé de manière que la bille 58 décolle du premier siège de valve 60 à une première pression d'ouverture prédéterminée P₁, permettant ainsi le passage de fluide à travers ce siège,
la bille 58 pouvant être déplacée axialement vers la restriction de passage 61', à l'encontre de l'effort exercé par le ressort 62, jusqu'à une position fermée d'obturation maximale, correspondant à une pression différentielle égale à une pression de fermeture P_{f} prédéterminée supérieure à P₁, où la bille 58 délimite avec la restriction de passage 61' une section de passage minimale pour la circulation du fluide à travers la restriction de passage 61',
la bille 58 pouvant encore être déplacée axialement au-delà de la restriction de passage lorsque la pression différentielle dépasse une pression d'ouverture à haute pression P_{HP} supérieure à la pression de fermeture P_{f}.

Le dispositif de drainage peut ainsi fournir une troisième valve à régulation de pression, s'ouvrant à une deuxième pression d'ouverture, égale à la pression d'ouverture à haute pression P_{HP}, et supérieure à la pression de fermeture P_{f}. Avec un tel dispositif, on réalise ainsi avantageusement, avec un unique obturateur, un système de drainage à trois valves tel que défini ci-dessus.

La pression d'ouverture P_{HP} est alors, de préférence, déterminée de manière à autoriser une augmentation du débit drainé lorsque la pression intracrânienne ou la pression hydrostatique augmente. La pression d'ouverture P_{HP} est de préférence ajustée en fonction des besoins du patient.

Dans un autre mode de réalisation, cette fonction d'augmentation du débit est assurée par une autre valve à régulation de pression, par exemple de type Polarisa® branchée en parallèle au dispositif de drainage. La pression d'ouverture à haute pression peut alors être déterminée pour ne pas être atteinte dans les conditions normales de service, mais seulement dans le cas d'opérations de nettoyage par chasse (« Flush »). Par exemple, la pression d'ouverture à haute pression peut être fixée à 1 m H₂O. L'application d'une pression différentielle suffisante pour que la bille 58 traverse la restriction de passage 61' permet avantageusement des opérations de nettoyage efficaces, simultanément de la restriction de passage et de la valve branchée en parallèle au dispositif de drainage, avec un risque réduit de colmatage par des déchets.

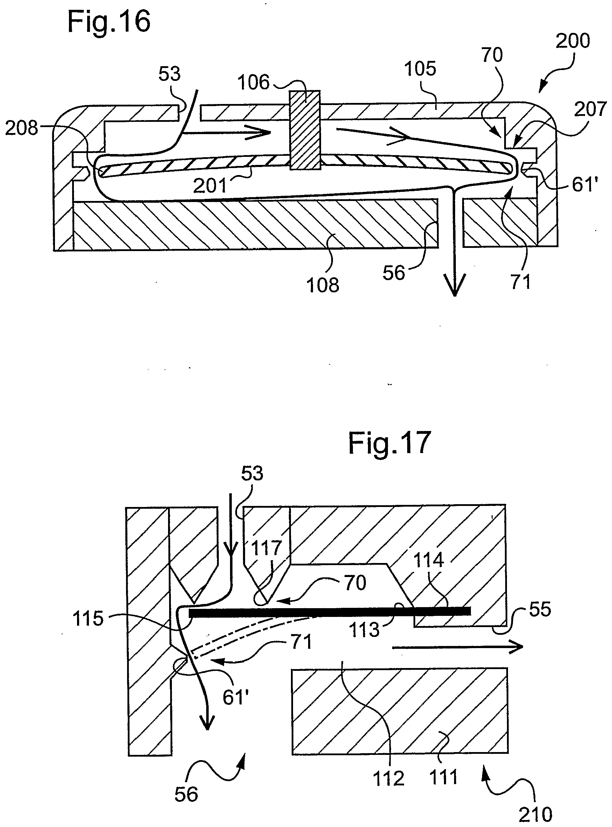

Dans ce dernier mode de réalisation, la valve branchée parallèlement au dispositif de drainage est de préférence réglable. La figure 16 représente un autre dispositif de drainage à obturateur traversant 200. Ce dispositif comporte une membrane élastiquement déformable 201 maintenue sur un corps 105 par l'intermédiaire d'un élément de maintien 106, formé par exemple d'une tige, fixée sur une portion centrale de la membrane 201. L'élément de maintien 106 peut être agencé pour pouvoir être réglable en translation afin de régler la première pression d'ouverture P₁ et la pression de fermeture P_{f} associées aux valves 70 et 71. Le corps 105 comporte un épaulement annulaire 207 servant de siège au bord périphérique 208 de la membrane 201 pour la première valve 70. Il comporte également une restriction de passage 61' que le bord périphérique 208 de la membrane 201 peut traverser lorsque la pression différentielle dépasse la pression de fermeture P_{f}.

Lorsque la pression différentielle est inférieure à une pression d'ouverture P₁, le bord périphérique 208 de la membrane 201 prend appui, de manière étanche, sur l'épaulement 207, isolant ainsi le port de sortie 56 du port d'entrée 53. La première valve 70 est alors fermée. Lorsque la pression différentielle dépasse P₁, la valve 70 s'ouvre. Le bord périphérique 208 de la membrane 201 vient ensuite obturer progressivement la restriction de passage 61', jusqu'à une position « fermée » d'obturation maximale de cette restriction de passage 61', comme représenté sur la figure 16. Au-delà de la pression de fermeture P_{f}, le bord périphérique 208 dégage la restriction de passage 61' en passant à l'aval de cette restriction.

La figure 17 représente encore un autre dispositif de drainage 210 à obturateur traversant. Le dispositif de drainage 210 comporte :
- un corps 111 définissant une chambre interne 112,
- un port d'entrée 53, un port de dérivation 55, et un port de sortie 56 communiquant avec la chambre interne 112,
- une lame élastiquement déformable comprenant une première portion 114 maintenue de manière fixe sur le corps 111 et une deuxième portion 115 mobile, formant obturateur.

Dans une position de repos, la portion 115 prend appui sur un premier siège 117 isolant ainsi le port d'entrée des ports de dérivation 55 et de sortie 56.

Lorsque la pression différentielle dépasse une première pression d'ouverture P₁, la portion mobile 115 décolle du siège 117, mettant ainsi en communication le port d'entrée 53 avec les ports de sortie 56 et de dérivation 55. Lorsque la pression différentielle se rapproche de la pression de fermeture P_{f}, le bord périphérique de la lame se rapproche d'une restriction de passage 61' ménagée sur le corps 110. Cette restriction est conformée de manière à ce que, lorsque la pression différentielle atteint une pression de fermeture P₁ (trait interrompu), la section de passage soit minimale. Au-delà de la position d'obturation maximale de la restriction de passage 61', représentée sur la figure 17, le bord périphérique de la lame 113 « traverse » la restriction de passage 61', autorisant ainsi une nouvelle augmentation du débit.

On a représenté sur la figure 2 un autre dispositif de drainage 80 comportant :
- un corps 81 définissant une chambre interne 52,
- un port d'entrée 53, un port de dérivation 55 et un port de sortie 56 communiquant avec la chambre interne 52,
- une membrane élastiquement déformable 82, notamment en élastomère, par exemple en silicone ou en polyuréthane, présentant une forme sensiblement de disque et comportant un passage central 83 agencé pour permettre le passage de fluide, la membrane 82 comportant en outre des lèvres inférieure 84 et supérieure 85 annulaires, bordant le passage central 83, la lèvre inférieure, et respectivement supérieure, s'étendant sur une face inférieure 86, respectivement supérieure 87, de la membrane 82,
- un premier siège de valve 90 sur lequel la lèvre supérieure 85 de la membrane 82 peut s'appliquer lorsque la membrane 82 est au repos, de manière à isoler le port d'entrée 53 des ports de dérivation 55 et de sortie 56,
- un deuxième siège de valve 91 sur lequel la lèvre inférieure 84 peut s'appliquer lorsque la membrane 82 se déforme suffisamment, afin d'isoler le port de sortie 56 des ports d'entrée 53 et de dérivation 55.

Les lèvres 84 et 85 présentent, en section transversale, par exemple une section triangulaire.

La membrane 82 est maintenue sur le corps 81 par l'intermédiaire d'une portion périphérique de cette membrane 82.

Les sièges de valve 90 et 91 sont réalisés par exemple en matière plastique ou en métal, et sont agencés de manière à pouvoir être réglables par coulissement afin de pouvoir régler, d'une part, le niveau de la première pression d'ouverture P₁ de la valve 70 formée par la lèvre supérieure 85 et le siège de valve 90 et, d'une part, le niveau de la pression de fermeture P_{f} de la deuxième valve 71 formée par la lèvre inférieure 84 et le siège 91.

On a représenté sur la figure 3 un autre dispositif de drainage 100 comportant, à l'instar du dispositif 80, une membrane élastiquement déformable 101 pourvue à sa périphérie de lèvres annulaires inférieure 102 et supérieure 103.

La membrane 101 est maintenue sur le corps 105 du dispositif 100, par l'intermédiaire d'un élément de maintien 106, formé par exemple d'une tige, fixé sur une portion centrale de la membrane 101.

L'élément de maintien 106 peut être agencé pour pouvoir être réglable en translation afin de régler les premières pressions d'ouverture P₁ et de fermeture P_{f} associées aux valves 70 et 71.

Les lèvres 102 et 103 présentent, en section transversale, par exemple une forme triangulaire et sont logées dans un renforcement annulaire 109 du corps 105.

Le corps 105 comporte une portion inférieure 108 définissant un siège de valve pour la lèvre 102, cette portion 108 pouvant être déplaçable en translation afin de régler la pression de fermeture P_{f} de la valve associée 71.

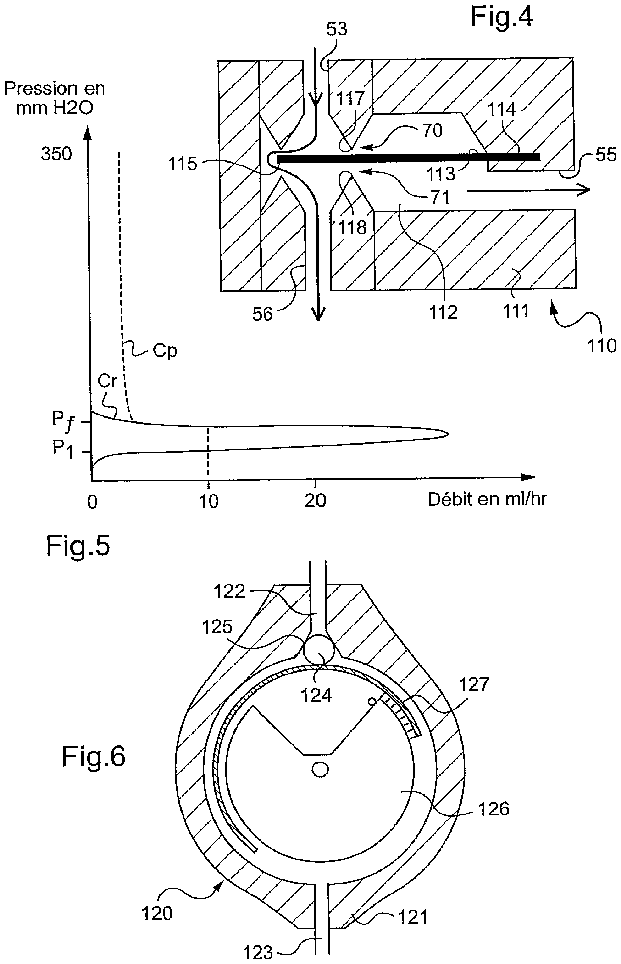

On a représenté sur la figure 4 un dispositif de drainage 110 comportant :
- un corps 111 définissant une chambre interne 112,
- un port d'entrée 53, un port de dérivation 55, et un port de sortie 56 communiquant avec la chambre interne 112,
- une lame élastiquement déformable 113 comprenant une première portion 114 maintenue de manière fixe sur le corps 111, et une deuxième portion 115 mobile entre une première, respectivement une deuxième, position dans laquelle la lame s'applique contre un premier siège 117, respectivement un deuxième siège 118, cette deuxième portion mobile 115 formant ainsi un obturateur au sens de l'invention.

Les sièges 117 et 118 sont formés par exemple par des portions de section transversale de forme sensiblement triangulaire.

Les sièges 117 et 118, réalisés par exemple en élastomère, peuvent être agencés pour pouvoir être déplaçables en translation afin de régler, d'une part, la première pression d'ouverture P₁ de la valve 70 formée par la portion mobile 115 de la lame 113 et le siège 117 et, d'autre part, la pression de fermeture P_{f} de la deuxième valve 71 formée par cette partie mobile 115 et le deuxième siège 118.

Lorsque la portion mobile 115 s'applique contre le siège 117, le port d'entrée 53 est isolé des ports de dérivation 55 et de sortie 56.

Lorsque la portion mobile 115 s'applique contre le deuxième siège 118, le port de sortie 56 est isolé des ports d'entrée 53 et de dérivation 55 qui communiquent entre eux.

Le graphique de la figure 5 illustre la variation de la pression en fonction du débit de fluide circulant dans l'un des dispositifs de drainage 50, 80, 100 et 110, dans des modes de réalisation à obturation totale (courbe C_{T}) ou partielle (courbe C_{P}) du siège de deuxième valve.

Les valeurs figurant sur ce graphique sont données à titre d'exemple, d'autres valeurs de débit et de pression pouvant bien entendu être utilisées.

De manière schématique, la première valve 70 s'ouvre lorsque la pression du fluide céphalorachidien atteint la première pression d'ouverture P₁.

Lorsque la pression du fluide atteint la pression de fermeture P_{f}, la valve 71 se ferme, totalement ou partiellement.

On a représenté sur la figure 6 un exemple de troisième valve à régulation de pression 120.

Cette valve 120 est décrite en détail dans le brevet US 4 673 384.

Cette valve 120 comporte un corps 121, par exemple en matière plastique, présentant un port d'entrée 122 et un port de sortie 123.

La valve 120 comporte un obturateur 124 formé par une bille s'appliquant sur un siège 125 du corps 121.

La valve 120 comporte en outre un rotor 126 portant une lame ressort 127 en arc de cercle s'appliquant sur la bille 124 afin de fermer le port d'entrée 122.

Le port d'entrée 122 peut être ouvert lorsque la bille 124 est soumise à une deuxième pression d'ouverture P₂ lui permettant de s'éloigner du siège 125 à l'encontre de l'effort exercé par la lame ressort 127.

La troisième valve à régulation de pression peut être de tout autre type, par exemple à membrane élastiquement déformable.

La troisième valve peut être du type réglable depuis l'extérieur à plusieurs niveaux de pression d'ouverture et peut comporter ou non un aimant.

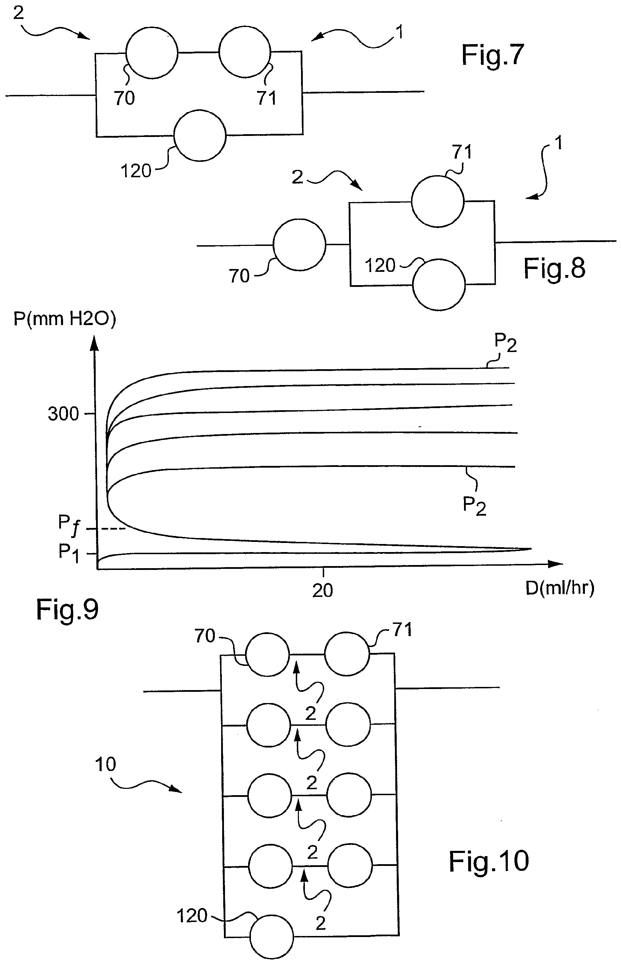

On a représenté sur la figure 7 un exemple de système de drainage 1 pour le traitement de l'hydrocéphalie, le système 1 comportant :
- un groupe 2 de valves comportant :
   - une première valve à régulation de pression 70 pouvant s'ouvrir à une première pression d'ouverture prédéterminée P₁ et permettre le passage de fluide,
   - une deuxième valve 71 pouvant se fermer totalement ou partiellement à une pression de fermeture prédéterminée P_{f} supérieure à la première pression d'ouverture P₁, les première et deuxième valves 70 et 71 étant agencées de manière à ce que du fluide puisse être drainé à travers la deuxième valve 71 lorsque la pression du fluide est comprise entre la première pression d'ouverture P₁ et la pression de fermeture P_{f},
- une troisième valve à régulation de pression 120 pouvant s'ouvrir à une deuxième pression d'ouverture P₂ supérieure à la pression de fermeture P_{f}, les deuxième et troisième valves 71 et 120 étant agencées de manière à ce que, lorsque la deuxième valve 71 est fermée, du fluide puisse être drainé à travers la troisième valve 120 et éventuellement, si la fermeture de la deuxième valve ne peut qu'être partielle, à travers cette deuxième valve.

On peut avoir par exemple : P₂ > 1,1 P_{f}.

Dans l'exemple illustré à la figure 7, les première et deuxième valves 70 et 71 sont disposées en série, la première valve 70 étant en amont de la deuxième valve 71, la troisième valve 120 étant disposée en parallèle avec l'ensemble formé des première et deuxième valves 70 et 71.

En variante, comme illustré sur la figure 8, les deuxième et troisième valves 71 et 120 sont disposées en parallèle, la première valve 70 étant disposée en série avec l'ensemble formé des deuxième et troisième valves 71 et 120, en amont de cet ensemble.

Les valves 70 et 71 sont formées par l'un des dispositifs 50, 80, 100 et 110.

Le graphique de la figure 9 illustre la variation du débit en fonction de la pression pour le système 1 décrit ci-dessus, la troisième valve à régulation de pression 120 étant agencée pour permettre d'obtenir différents niveaux de pression d'ouverture P₂.

Comme expliqué précédemment, un système de drainage à trois valves peut également être réalisé avec un unique obturateur apte à traverser une restriction de passage de deuxième valve, comme illustré par exemple sur les figures 13a à 13c, 16 et 17.

Lorsque les valves 70 et 71 sont formées par le dispositif de drainage 50 et le système 1 est dans la configuration de la figure 7, seul le port de dérivation 54 est utilisé, lequel est connecté à la troisième valve 120.

Dans la configuration de la figure 8, seul le port de dérivation 55 est utilisé, lequel est connecté à la valve 120.

On a représenté sur la figure 10 un système de drainage 10, comportant une pluralité de groupes 2 de valves formés chacun d'une première valve à régulation de pression 70 et d'une deuxième valve 71.

Dans l'exemple illustré à la figure 10, les groupes 2 et la troisième valve à régulation de pression 120 sont disposés tous en parallèle.

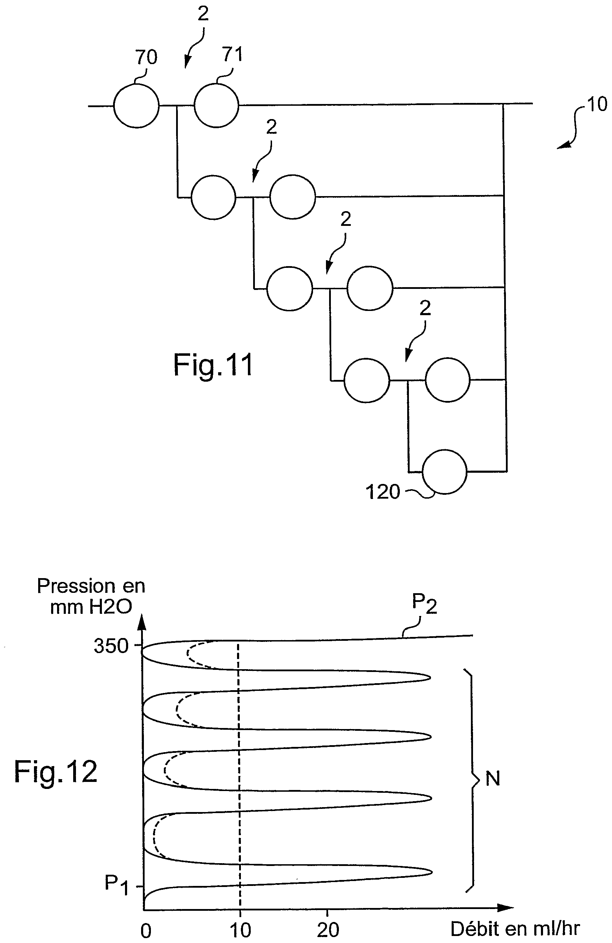

En variante, comme illustré sur la figure 11, les valves 71 et 72 d'un groupe 2 sont disposées de manière à ce que le port d'entrée de ce groupe 2 soit connecté au port de dérivation d'un autre des groupes 2 de valves.

La troisième valve 120 est connectée au port de dérivation de l'un des groupes de valves.

Le graphique de la figure 12 illustre la variation de la pression en fonction du débit pour le système de drainage 10. Le trait interrompu correspond à cette variation lorsque les deuxièmes valves 71 autorisent, dans la position fermée d'obturation maximale, un débit minimum de sécurité d'environ 2,5 ml/h.

Le système 10 permet d'obtenir N niveaux de pression d'ouverture et de fermeture, grâce aux groupes 2 de valves.

Ainsi, la troisième valve à régulation de pression 120 peut, si on le souhaite, être du type monopression.

L'expression « comportant un » doit être comprise comme étant synonyme de « comportant au moins un », sauf si le contraire est spécifié.

Bien entendu, l'invention n'est pas limitée aux modes de réalisation décrits et représentés. En particulier, les caractéristiques préférées du ressort, de la membrane ou de la lame, ou des moyens de réglages décrits dans le cadre d'un dispositif de drainage à fermeture totale pourraient être appliquées avec un dispositif de drainage à obturation partielle.

## Revendications

1. Dispositif de drainage, comportant :
- une chambre interne (52),
- au moins un port d'entrée (53) et un port de sortie (57) communiquant avec la chambre interne,
- au moins un obturateur (58) disposé au moins partiellement dans la chambre interne,
- au moins un premier siège de valve (60) associé au port d'entrée, l'obturateur pouvant s'appliquer sur le premier siège afin d'isoler le port d'entrée du port de sortie, notamment lorsque la pression différentielle entre les ports d'entrée et de sortie est inférieure à une première pression d'ouverture prédéterminée,
- au moins une restriction de passage (61') associée au port de sortie, l'obturateur pouvant obturer ladite restriction, en autorisant un débit minimum de sécurité,
le dispositif étant **caractérisé en ce que** le débit minimum de sécurité est supérieur ou égal à 2,5 ml/h même dans une position d'obturation maximale, dite « position fermée », notamment atteinte pour des pressions différentielles entre les ports d'entrée et de sortie supérieures ou égales à une pression de fermeture prédéterminée, et **en ce que** l'obturateur et la restriction de passage sont conformés de manière à ce que l'obturateur puisse traverser la restriction de passage lorsque la pression différentielle dépasse une pression d'ouverture à haute pression supérieure à ladite pression de fermeture.

2. Dispositif de drainage selon la revendication précédente, dans lequel l'obturateur comporte une bille (58), un ressort de rappel (62) étant agencé pour exercer sur la bille un effort tendant à l'appliquer sur le premier siège, la bille étant agencée pour pouvoir être déplacée vers ladite restriction de passage, à l'encontre de l'effort exercé par le ressort, le ressort comportant au moins une portion de base tronconique (64) s'élargissant vers l'extrémité inférieure du ressort et une portion supérieure cylindrique (63) se raccordant à ladite portion de base tronconique.

3. Dispositif de drainage selon l'une quelconque des revendications précédentes, dans lequel la chambre interne présente en amont de la restriction de passage, la forme d'un tronc de cône s'évasant vers l'amont de l'écoulement, et/ou dans lequel la chambre interne se prolonge, en aval de la restriction de passage, par un tronc de cône (14') s'évasant vers l'aval de l'écoulement, d'angle au sommet (3 supérieur à l'angle au sommet a du tronc de cône amont (10').

4. Dispositif de drainage selon l'une quelconque des revendications précédentes, conformé de manière à ce que, lorsque la pression différentielle augmente, ledit obturateur ne commence à diminuer le débit à travers le premier siège de valve que si ce débit est compris entre 15 et 20 ml/h.

5. Dispositif de drainage selon l'une quelconque des revendications précédentes comportant un port de dérivation communiquant avec la chambre interne, les ports d'entrée et de dérivation (55) communicant entre eux et étant partiellement isolés du port de sortie par obturation de la restriction de passage par l'obturateur.

6. Dispositif de drainage selon l'une quelconque des revendications précédentes, ladite pression d'ouverture à haute pression étant au moins 10% supérieure à ladite pression de fermeture.

7. Dispositif de drainage selon la revendication précédente, dans lequel ladite deuxième pression d'ouverture à haute pression est au moins 10 % supérieure à ladite pression de fermeture, le dispositif étant conformé de manière à empêcher un débit de drainage supérieur à 20ml/h si la pression différentielle ne dépasse pas ladite pression d'ouverture à haute pression et/ou à garantir un débit minimum de sécurité d'au moins 5 ml/h quand la pression différentielle atteint ou dépasse ladite pression de fermeture.

8. Dispositif de drainage selon l'une quelconque des revendications précédentes dans lequel la première pression d'ouverture et/ou la pression de fermeture et/ou, le cas échéant, la pression d'ouverture à haute pression est ou sont réglables, ce réglage pouvant notamment être réalisé sans contact, le dispositif comportant soit un moteur permettant d'effectuer le ou les réglages et recevant ses instructions de commande et/ou son énergie par l'intermédiaire de radiofréquences, soit des moyens permettant un couplage magnétique avec l'extérieur du corps du patient pour effectuer le ou les réglages.

9. Dispositif de drainage selon l'une quelconque des revendications précédentes, dans lequel la restriction de passage constitue un deuxième siège de valve sur lequel l'obturateur peut venir s'appliquer, l'obturateur et le deuxième siège de valve formant une deuxième valve pouvant se fermer, en autorisant toujours un débit minimum de sécurité supérieur ou égal à 2,5 ml/h, à une pression de fermeture prédéterminée supérieure à la première pression d'ouverture, le dispositif comportant au moins une membrane élastiquement déformable (82) avec au moins une portion définissant l'obturateur, la membrane élastiquement déformable comportant :
- un passage central (83) agencé pour permettre le passage de fluide,
- des lèvres inférieure (84) et supérieure (85), bordant sensiblement le passage central, la lèvre inférieure, respectivement supérieure, s'étendant sur une face inférieure, respectivement supérieure, de la membrane, la lèvre inférieure, respectivement supérieure, pouvant s'appliquer sur l'un des sièges de valve, respectivement l'autre des sièges de valve, en fonction de la déformation de la membrane, les lèvres définissant ainsi l'obturateur.

10. Dispositif de drainage selon l'une quelconque des revendications précédentes, dans lequel l'obturateur et le bord de la restriction de passage sont conformés de manière à définir, dans la position d'obturation maximale, une section de passage entre l'obturateur et le bord de la restriction de passage telle que le débit minimum de sécurité, dans cette position, soit supérieur à 5ml/h et inférieur à 20 ml/h.

## Patentansprüche

1. Drainagevorrichtung, umfassend:
- eine innere Kammer (52),
- mindestens eine Zulauföffnung (53) und eine Auslauföffnung (57), die mit der inneren Kammer verbunden sind,
- mindestens einen Verschlusskörper (58), der zumindest teilweise in der inneren Kammer angeordnet ist,
- mindestens einen ersten Ventilsitz (60), der mit der Zulauföffnung verbunden ist, wobei der Verschlusskörper in Verbindung mit dem ersten Ventilsitz wirksam werden kann, um die Zulauföffnung gegenüber der Auslauföffnung abzusperren, insbesondere dann, wenn der Differentialdruck zwischen Zulauf- und Auslauföffnung kleiner ist als ein erster vorgegebener Öffnungsdruck,
- mindestens eine Durchflussverengung (61'), die mit der Auslauföffnung verbunden ist, wobei der Verschlusskörper die Verengung unter Aufrechterhaltung eines sicherheitsbedingten Mindestdurchflusses absperren kann,
wobei die Vorrichtung **dadurch gekennzeichnet ist, dass** ein sicherheitsbedingter Mindestdurchfluss selbst bei maximaler Schließung größer bzw. gleich 2,5 ml/h ist, wobei die "geschlossene Position" insbesondere bei Differenzdrücken zwischen Ein-und Auslauföffnung, die größer bzw. gleich einem vorgegebenen Schließdruck sind, erreicht wird, und dass der Verschlusskörper und die Durchflussverengung in der Weise aufeinander abgestimmt sind, dass sich der Verschlusskörper durch die Durchflussverengung hindurch bewegen kann, wenn der Differenzdruck einen Hochdruck-Öffhungsdruck überschreitet, der größer ist als der Schließdruck.

2. Drainagevorrichtung nach dem vorhergehenden Anspruch, wobei der Verschlusskörper eine Kugel (58, eine Rückstellfeder (62) umfasst, wobei letztere so angeordnet ist, dass sie zwecks Ansteuerung des ersten Ventilsitzes auf die Kugel eine Kraft ausüben kann, wobei die Kugel so angeordnet ist, dass sie sich in Richtung der Durchflussverengung gegen die von der Feder ausgeübte Kraft verschieben lässt, wobei die Feder mindestens einen kegelstumpfförmigen unteren Bereich (64) aufweist, der zum unteren Ende der Feder hin breiter wird, sowie einen oberen zylindrischen Bereich (63), der sich an den unteren Bereich anschließt.

3. Drainagevorrichtung nach einem der vorhergehenden Ansprüche, wobei die innere Kammer hinter der Durchflussverengung die Form eines Kegelstumpfes hat, der sich bis zu dem Abschnitt vor dem Auslauf verbreitert, und/oder wobei sich die innere Kammer bis zu dem Abschnitt hinter der Durchflussverengung über einen Kegelstumpf (14') verlängert, der sich bis zu dem Abschnitt vor dem Auslauf in einem Winkel zum Scheitelpunkt β, der größer ist, als der Winkel zum Scheitelpunkt α des davor befindlichen Kegelstumpfes (10'), verbreitert.

4. Drainagevorrichtung nach einem der vorhergehenden Ansprüche, die so angepasst ist, dass bei Erhöhung des Differenzdrucks der Schließvorgang erst dann bei der Verringerung des Durchflusses vermittels des ersten Ventilsitzes einsetzt, wenn dieser Durchfluss zwischen 15 und 20 ml/h beträgt.

5. Drainagevorrichtung nach einem der vorhergehenden Ansprüche, die eine Bypass-Öffnung aufweist, welche mit der inneren Kammer verbunden ist, wobei die Zulauföffnung und die Bypass-Öffnung (55) miteinander verbunden und teilweise von der Auslauföffnung durch Verschließen der Durchflussverengung durch den Verschlusskörper voneinander getrennt sind.

6. Drainagevorrichtung nach einem der vorhergehenden Ansprüche, wobei der Hochdruck-Öffnungsdruck mindestens 10% über dem Schließdruck liegen muss.

7. Drainagevorrichtung nach einem der vorhergehenden Ansprüche, wobei der zweite Hochdruck-Öffnungsdruck mindestens 10 % höher ist als der Schließdruck, und die Vorrichtung so angepasst ist, dass ein Drainagedurchfluss von mehr als 20ml/h verhindert wird, wenn der Differenzdruck den Hochdruck-Öffnungsdruck nicht überscheitet und/oder dass ein sicherheitsbedingter Mindestdurchfluss von mindestens 5 ml/h gewährleistet wird, wenn der Differenzdruck den Schließdruck erreicht oder überschreitet.

8. Drainagevorrichtung nach einem der vorhergehenden Ansprüche, wobei der erste Schließdruck und/oder der Schließdruck und/oder ggf. der Hochdruck-Öffnungsdruck einstellbar ist oder sind, wobei die Einstellung vor allem berührungsfrei erfolgen kann, wobei die Vorrichtung entweder einen Motor umfasst, mit dem die Einstellung(en) vorgenommen werden kann/können, und welcher seine Steuerbefehle und/oder seine Energieversorgung über Funkfrequenzen erhält, oder Mittel, die eine magnetische Kopplung über die Außenseite des Körpers des Patienten zwecks Vornahme der Einstellung(en) ermöglichen.

9. Drainagevorrichtung nach einem der vorhergehenden Ansprüche, wobei die Durchflussverengung einen zweiten Ventilsitz bildet, auf den der Verschlusskörper einwirken kann, wobei der Verschluss und der zweite Ventilsitz ein zweites Ventil bilden, das sich schließen und damit einen sicherheitsbedingten Mindestdurchfluss sicherstellen kann, der größer oder gleich 2,5 ml/h ist, bei einem vorgegebenen Schließdruck, der höher ist als der erste Öffnungsdruck, wobei die Vorrichtung mindestens eine elastisch verformbare Membran (82) mit mindestens einem Abschnitt aufweist, der den Verschluss definiert, wobei die elastisch verformbare Membran umfasst:
- eine mittig angeordnete Durchführung (83), die den Durchfluss der Flüssigkeit ermöglicht,
- untere (84) und obere Lippen (85), die im Wesentlichen an die mittig angeordnete Durchführung grenzen, wobei die untere bzw. die obere Lippe sich über eine untere bzw. obere Fläche der Membran erstrecken, und die untere bzw. die obere Lippe auf einen der beiden Ventilsitze in Abhängigkeit von der Verformung der Membran einwirken kann, und die Lippen somit den Verschluss definieren.

10. Drainagevorrichtung nach einem der vorhergehenden Ansprüche, wobei der Verschlusskörper und der Rand der Durchflussverengung so angepasst sind, dass in der maximalen Schließposition zwischen dem Verschlusskörper und dem Rand der Durchflussverengung ein Durchflussquerschnitt erhalten bleibt, so dass ein sicherheitsbedingter Mindestdurchfluss in dieser Position von mehr als 5ml/h und weniger als 20 ml/h gewährleistet ist.

## Claims

1. A drainage device comprising:
• an internal chamber (52);
• at least one inlet port (53) and at least one outlet port (57) communicating with the internal chamber;
• at least one shutter (58) disposed at least in part in the internal chamber;
• at least one first valve seat (60) associated with the inlet port, the shutter being configured to bear against the first seat in order to isolate the inlet port from the outlet port, in particular when the pressure difference between the inlet and outlet ports is less than a predetermined first opening pressure; and
• at least one flow constriction (61') associated with the outlet port, the shutter being configured to shut said constriction, while allowing a minimum safety flow, the device being **characterized in that** the minimum safety flow is greater than or equal to 2.5 mL/h even in a maximally-shut position, referred to as a "closed" position, which position is in particular reached for pressure differences between the inlet and outlet ports that are greater than or equal to a predetermined closure pressure, and **in that** the shutter and the flow constriction are shaped in such a manner that the shutter can pass through the flow constriction when the pressure difference exceeds a high-pressure opening pressure greater than said closure pressure.

2. The drainage device according to the preceding claim, in which the shutter comprises a ball (58), a return spring (62) arranged to exert a force on the ball urging it against the first seat, the ball being arranged to be movable towards said flow constriction against the force exerted by the spring, the spring including at least a frustoconical base portion enlarging towards a bottom end of the spring, and a cylindrical top portion (63) connected to said frustoconical base portion.

3. The drainage device according to any one of the preceding claims, in which the internal chamber presents a frustoconical shape upstream from the flow constriction, the frustoconical shape flaring upstream relative to a flow direction and/or, in which the internal chamber is extended downstream from the flow constriction by a truncated cone (14') extending downstream in the flow direction, with a half-angle at an apex β greater than a half-angle at an apex α of the upstream truncated cone (10').

4. The drainage device according to any one of the preceding claims, shaped in such a manner that when the pressure difference increases, said shutter begins to reduce a flow rate through the first valve seat only if said flow rate lies in a range of from 15 mL/h to 20 mL/h.

5. The drainage device according to claim 1, further comprising a branch port communicating with the internal chamber, the inlet and branch ports (55) communicating with each other and being partially isolated from the outlet port by the shutter shutting the flow constriction.

6. The drainage device according to any one of the preceding claims, said high-pressure opening pressure being at least 10% greater than said closure pressure.

7. The drainage device according to the preceding claim, in which said high-pressure second opening pressure is at least 10% greater than said closure pressure, the device being shaped in such a manner as to prevent a drainage flow rate greater than 20 mL/h if the pressure difference does not exceed said high-pressure opening pressure, and/or to guarantee a safety minimum flow rate of at least 5 mL/h when the pressure difference reaches or exceeds said closure pressure.

8. The drainage device according to any one of the preceding claims, in which the first opening pressure and/or the closure pressure and/or, where appropriate, a high-pressure opening pressure is or are adjustable, it being possible to perform said adjustment without contact, this device comprising either:
a motor configured to enable the adjustment(s), the motor being configured to receive control instructions and/or power via radiofrequency waves, or
means configured to enable magnetic coupling with an outside of a patient's body in order to perform the adjustment(s).

9. The drainage device according to any one of the preceding claims, in which the flow constriction constitutes a second valve seat against which the shutter can bear, the shutter and the second valve seat forming a second valve that is configured to close at a predetermined closure pressure greater than the first opening pressure, while continuing to allow a safety minimum flow to pass at a rate greater than or equal to 2.5 mL/h, the device comprising at least one elastically deformable diaphragm (82) having at least a portion that defines the shutter, the elastically deformable diaphragm including:
• a central passage (83) arranged to allow fluid to pass through; and
• bottom (84) and top (85) lips substantially adjacent to the central passage, the bottom and top lips extending respectively from bottom and top faces of the diaphragm, the bottom and top lips configured to bear against respective ones of the valve seats as a function of a deformation of the diaphragm, the lips thus defining the shutter.

10. The drainage device according to any one of the preceding claims, in which the shutter and an edge of the flow constriction are shaped in such a manner as to define, in the maximally-shut position, a flow section between the shutter and the edge of the flow constriction such that the safety minimum flow rate in this position is greater than 5 mL/h, and less than 20 mL/h.
